(19) Europäisches Patentamt
European Patent Office
Office européen des brevets

(11) **EP 1 287 810 B1**

(12) **EUROPÄISCHE PATENTSCHRIFT**

(45) Veröffentlichungstag und Bekanntmachung des Hinweises auf die Patenterteilung:
**02.11.2005 Patentblatt 2005/44**

(51) Int Cl.⁷: **A61K 7/06**, A61K 7/11, A61K 7/13

(21) Anmeldenummer: **02012961.5**

(22) Anmeldetag: **12.06.2002**

(54) **Itaconsäuremonoester/Acrylat Copolymer enthaltendes, gelförmiges Haarbehandlungsmittel**

Hair gel comprising itaconic acid monoester acrylates copolymer

Gel pour traitement des cheveux comprenant copolymère acide itaconique monoester/acrylat

(84) Benannte Vertragsstaaten:
**AT BE CH CY DE DK ES FI FR GB GR IE IT LI LU MC NL PT SE TR**

(30) Priorität: **31.08.2001 DE 10142476**

(43) Veröffentlichungstag der Anmeldung:
**05.03.2003 Patentblatt 2003/10**

(73) Patentinhaber: **Wella Aktiengesellschaft 64274 Darmstadt (DE)**

(72) Erfinder:
• **Birkel, Susanne, Dr.**
  **64285 Darmstadt (DE)**
• **Wendel, Harald**
  **64372 Ober-Ramstadt (DE)**
• **Franzke, Michael, Dr.**
  **64380 Rossdorf (DE)**
• **Niesig, Silke**
  **64401 Gross-Bieberau (DE)**

(56) Entgegenhaltungen:
WO-A-02/00177     WO-A-99/37278
WO-A-99/39688     DE-A- 19 816 662
US-A- 5 972 356     US-A1- 2002 042 448

• **NATIONAL STARCH & CHEMICAL * PERSONAL CARE: "Modify rheology: STRUCTURE 2001/STRUCTURE 3001" [Online] XP002209084 Gefunden im Internet: <URL: http://www.personalcarepolymers.com/Site/List.asp?ID=Enhance.7> [gefunden am 2002-08-08] * technical bulletin * * formulations ***
• **PATENT ABSTRACTS OF JAPAN vol. 1998, no. 11, 30. September 1998 (1998-09-30) & JP 10 158129 A (KANEBO LTD), 16. Juni 1998 (1998-06-16)**

**Beschreibung**

Gegenstand der Erfindung ist ein Haarbehandlungsmittel in Gelform mit einem Gehalt an mindestens einem Itaconsäuremonoester/Acrylat Copolymer, haarfestigenden, filmbildenden Polymeren und mit bestimmten rheologischen Eigenschaften.

Haargele werden eingesetzt, um dem menschlichen Haar Festigung und Halt zu geben. Übliche Haargele enthalten in der Regel eine Kombination aus Gelbildnern und haarfestigenden Polymeren. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung mehr oder weniger gut die Haare in Form halten und festigen. Häufig ist damit aber ein unbefriedigender Glanz des Haares verbunden. Es sind zwar eine Reihe von Additiven bekannt, welche in der Lage sind, den Haarglanz zu verbessern, z.B. hydrophobe Stoffe wie flüssige Paraffine, Isoparaffine, Silikonöle oder hydrophile Stoffe wie mehrwertige Alkohole, insbesondere Glycerin oder Propylenglykol. Die bekannten Glanzgeber wirken sich aber häufig nachteilig auf andere, erwünschte Eigenschaften eines Haargels aus. Sie können als Weichmacher für das verwendete festigende Polymer wirken und so die Festigungsleistung herabsetzen, sie können inkompatibel mit der wässrigen Gelbasis sein, sie können die Klarheit und Transparenz des Gels, das rheologische Verhalten und damit die Anwendungseigenschaften beeinträchtigen oder sie sind in ihrer Haarglanz erzeugenden Wirkung noch nicht ausreichend.

Eine weitere wichtige Eigenschaft von Haargelen ist ihr rheologisches Verhalten. Gele sind einerseits durch eine hohe Viskosität charakterisiert, andererseits durch die Eigenschaften von nicht-newtonschen Flüssigkeiten, insbesondere die Eigenschaften von pseudoplastischen Flüssigkeiten mit oder ohne Fließgrenze. Hierbei nimmt die Viskosität mit steigender Schergeschwindigkeit ab (shear thinning) und die Fließkurve ist nicht linear, d.h. das Verhältnis von Scherspannung und Schergeschwindigkeit ist nicht konstant. Dies macht sich praktisch dadurch bemerkbar, dass ein Gel in der Ruhephase eine relativ hohe Viskosität aufweist bzw. plastisches Verhalten zeigt, durch die Anwendung von relativ geringen Kräften aber fließfähig wird und damit beispielsweise leicht aus der Verpackung entnommen werden sowie leicht mit den Händen verrieben und leicht im Haar verteilt werden kann. Typische und häufig verwendete Gelbildner, welche Gele mit unter Druck abnehmender Viskosität bilden, sind neutralisierte Carbomere (Polyacrylsäure).

Der Nachteil von herkömmlichen Haargelen besteht darin, dass aufgrund der durch das Eigengewicht des Mittels hervorgerufenen Scherbeanspruchung die Viskosität abnimmt und das Gel fließfähig werden kann, wobei das Mittel aus einer nach unten gehaltenen, offenen Tube auch ohne externen Druck heraustropfen kann oder nach manuellem Auftragen auf senkrechte oder schräge Haut- oder Haaroberflächen unerwünschterweise herablaufen kann, im Falle von thixotropem Verhalten sogar mit zunehmender Geschwindigkeit. Unerwünschtes Abtropfen und Herablaufen kann natürlich durch Erhöhung der Basisviskosität vermieden werden, dadurch werden aber gleichzeitig die Anwendungseigenschaften wie z.B. die Verreibbarkeit und die Einarbeitbarkeit ins Haar beeinrächtigt und es ist eine höhere Einsatzmenge an Gelbildner erforderlich, was zur Verteuerung des Produkts und zu einer erhöhten Belastung des Haares führen kann.

In der Produktinformation "Structure® 2001/Structure® 3001" .., von National Starch & Chemical (www.personalcarepolymers.com/Site/List.asp?ID=Enhance.7) werden kosmetische Produkte beschrieben mit einem Gehalt an den Verdickern Acrylates/Steareth-20 Itaconate Copolymer und Acrylates/Ceteth-20 Itaconate Copolymer. In der US 5,972,356 wird in Beispiel XI eine Gelzusammensetzung beschrieben mit einem Gehalt an einem Acrylate Dimethicone Copolymer in Kombination mit einem Acrylates Steareth Itaconate Copolymer. In der WO 99/37278 werden in den Beispielen 1 und 2 oxidative Haarfärbemittel beschrieben, welche aus zwei Komponenten bestehen, wobei eine der Komponenten ein kationisches Polymer enthält und die zweite Komponente ein Acrylates/Steareth-20 Itaconate Copolymer und ein Acrylates/Ceteth-20 Itaconate Copolymer enthält. In der WO 99/39688 wird in Tabelle 1 ein Haargel beschrieben, welches ein amphoteres Urethanharz und Acrylates/Steareth-20 Itaconate Copolymer (Structure® 2001) enthält. Die WO 02/00177 beschreibt Mittel zur oxidativen Färbung keratinischer Fasern mit einem Gehalt an Acrylates/Steareth-20 Itaconate Copolymer (Structure® 2001).

Die Aufgabe der vorliegenden Erfindung bestand darin, die oben genannten Nachteile herkömmlicher Haargele zu vermeiden, insbesondere ein Haargel zur Verfügung zu stellen, welches einerseits eine gute Haarfestigung bei gleichzeitig optimiertem Haarglanz bewirkt, andererseits die rheologischen Nachteile bekannter Haargele vermeidet sowie attraktivere, angenehmere haptische Eigenschaften aufweist.

Es wurde nun gefunden, dass die Aufgabe gelöst wird durch ein Haarbehandlungsmittel in Gelform auf wässriger Basis mit einer Viskosität von mindestens 1000 mPa s bei 25°C, einem pH-Wert größer als 7 und mit einem Gehalt an einer Kombination von

(A) mindestens 0,5 Gew.% eines Copolymeren aus einer ersten Monomerart, ausgewählt aus Itaconsäuremonoestern der allgemeinen Formel $CH_2=C(COOR^1)CH_2COOR^2$ wobei einer der Substituenten $R^1$ und $R^2$ für Wasserstoff und der andere für die Gruppe $-(CH_2CH_2O)_x-R^3$ steht, x eine Zahl zwischen 1 und 100 bedeutet und $R^3$ eine

Cetylgruppe bedeutet und einer zweiten Monomerart, ausgewählt aus Acrylat-Monomeren und

(B) einem Gehalt an mindestens 0,1 Gew.% mindestens eines haarfestigenden nichtionischen, anionischen, zwitterionischen oder amphoteren Polymeren,

ausgenommen sein kann ein Gel mit einem Gehalt an einer Kombination von (a) Acrylates/Ceteth-20-itaconat Copolymer,

(b) Celluloseacetatphtalat und

(c) HO- $(CH_2CH_2O)_{75}$-$(CH(CH_3)CH_2O)_{30}$-$(CH_2CH_2O)_{75}$-H.

Das Copolymer (A) wird vorzugsweise in einer Menge von 0,5 bis 20, besonders bevorzugt von 1,5 bis 10 Gew.% und das haarfestigende Polymer (B) in einer Menge von vorzugsweise 0,1 bis 15, besonders bevorzugt von 0,5 bis 10 Gew.% eingesetzt. Das erfindungsgemäße Haargel ist vorzugsweise im wesentlichen frei von kationischen Polymeren, d.h. es enthält entweder keine oder weniger als 0,02 Gew.% an kationischen Polymeren. Das erfindungsgemäße Haargel bewirkt eine gute Haarfestigung und einen verbesserten Haarglanz bei gleichzeitig verbessertem Fließverhalten und einer besonders angenehmen, einzigartigen Haptik.

Copolymer (A)

Das Copolymer (A) ist aufgebaut aus Itaconsäuremonoestern der allgemeinen Formel $CH_2$=C(COOR[1]) $CH_2COOR^2$ wobei einer der Substituenten R[1] und R[2] für Wasserstoff und der andere für die Gruppe - -$(CH_2CH_2O)_x$-R[3] steht. X ist eine Zahl zwischen 1 und 100 vorzugsweise zwischen 10 und 40, besonders bevorzugt 20. R[3] ist eine Cetylgruppe.

Die Acrylat-Monomeren des Copolymers (A) sind vorzugsweise ausgewählt aus Acrylsäure, Methacrylsäure und deren einfachen Estern, insbesondere den Acrylsäurealkylestern und Methacrylsäurealkylestern mit 1 bis 10, vorzugsweise 1 bis 4 C-Atomen in der Alkylgruppe. Geeignete Copolymere sind beispielsweise Acryl- oder Methacrylsäure/Itaconsäurepolyethoxycetylester Copolymere (INCI-Bezeichnungen: Acrylates/Ceteth-20 Itaconate Copolymer), wie sie von der Firma National Starch/ USA unter der Bezeichnung Structure® 3001 vertrieben werden. Die Säuregruppen sind in den eingesetzten Polymeren vorzugsweise durch organische oder anorganische Basen zu 50 bis 100% neutralisiert. Geeignete Neutralisationsmittel sind primäre oder sekundäre Amine, insbesondere Aminoalkanole mit vorzugsweise 1 bis 10 C-Atomen und 1 bis 3 Hydroxygruppen wie z.B. Aminomethylpropanol (AMP), Triethanolamin, Tetrahydroxypropylethylendiamin oder Monoethanolamin, aber auch Ammoniak, NaOH, KOH u.a..

Haarfestigendes Polymer (B)

Das haarfestigende Polymer (B) kann nichtionisch, anionisch, zwitterionisch oder amphoter sein ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar eine haarfestigende Wirkung zu erzielen.

Geeignete synthetische, nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Dialkylaminoalkylmethacrylamid, Dialkylaminoalkylacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)-acrylamid, Polyacrylamide, Polyvinylalkohole, sowie haarfestigende Polyethylenglykol/Polypropylenglykol Copolymere. Besonders bevorzugte nichtionische Polymere sind Polyvinylpyrrolidon und Polyvinylpyrrolidon/Vinylacetat Copolymere.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -$SO_3H$, -$OSO_3H$, -$OPO_2H$ und -$OPO_3H_2$, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist und wie sie z.B. unter dem Handelsnamen Amphomer® oder Amphomer® LV-71 der Firma NATIONAL STARCH, USA erhältlich sind.

Weitere Beispiele für geeignete haarfestigende Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten, oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43). Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Das erfindungsgemäße Mittel wird bevorzugt in einem wässrigen, einem alkoholischen oder in einem wässrig-alkoholischen Medium mit vorzugsweise mindestens 10 Gew.% Wasser und vorzugsweise maximal 40 Gew.% Alkohol konfektioniert. Als Alkohole können insbesondere die für kosmetische Zwecke üblicherweise verwendeten niederen Monoalkohole mit 1 bis 4 Kohlenstoffatomen wie z.B. Ethanol und Isopropanol enthalten sein. Das erfindungsgemäße Mittel weist einen pH-Wert von größer als 7, vorzugsweise von mindestens 7,3 auf. Bei geringeren pH-Werten ist die Neutralisierung der Säuregruppen des Itaconsäuremonoester Copolymers (A) nicht ausreichend und die Konsistenz ist zu dünn. Besonders bevorzugt ist der pH-Bereich zwischen 7,5 und 8,5. In einer bevorzugten Ausführungsform enthält das Gel zur weiteren Glanzverbesserung mehrwertige Alkohole, vorzugsweise solche mit 2 bis 5 C-Atomen und mit 2 bis 5 Hydroxygruppen in einer Menge von 0,1 bis 15, bevorzugt von 1 bis 10 Gew.%. Besonders bevorzugt sind Glycerin, Ethylenglykol und Propylenglykol, insbesondere 1,2-Propylenglykol.

Rheologisches Verhalten

Das erfindungsgemäße Mittel zeichnet sich durch ein besonders vorteilhaftes, spezielles rheologisches Verhalten aus, wodurch sowohl die Anwendungseigenschaften als auch die Haptik verbessert werden. Ein Gelstrang reißt bei der Entnahme aus der Verpackung "trocken" ab, ohne ein Nachlaufen oder Fädenziehen. Unbeabsichtigtes Herauslaufen aus einer Tube und Herablaufen an schrägen Oberflächen, wie z.B. von der Hand und vom Haar ist minimiert. Physikalisch ist das erfindungsgemäße Gel gekennzeichnet durch das Auftreten eines Maximums in der Viskositätskurve bei ansteigender Schergeschwindigkeit. Das Maximum liegt typischerweise bei Schergeschwindigkeiten im Bereich von 0,1 bis 5,0, vorzugsweise von 0,15 bis 2,0 s$^{-1}$ bei einer Temperatur von 25°C. Die Fließkurve weist bei der Scherung, bei der die Viskosität ein Maximum erreicht, einen Wendepunkt auf. Eine weitere Besonderheit ist, dass sich die Viskositätskurve für ansteigende Schergeschwindigkeit mit derjenigen für fallende Schergeschwindigkeiten kreuzt. Der Kreuzungspunkt liegt typischerweise bei Schergeschwindigkeiten im Bereich von 0,05 bis 3, vorzugsweise 0,1 bis 2 s$^{-1}$. Bei hohen Schergeschwindigkeiten liegt die Hinkurve oberhalb der Rückkurve und das Gel ist thixotrop. Bei niedrigen Schergeschwindigkeiten liegt die Hinkurve unterhalb der Rückkurve und das Gel ist anti-thixotrop. Gemessen werden können die Viskositäts- und Fließkurven beispielsweise mit einem Rheometer Bohlin CS System, wobei unter isothermen Bedingungen (25°C) die Schergeschwindigkeit mit linearer Geschwindigkeit von 0 bis auf einen Maximalwert z.B. ca. 100 s$^{-1}$ gesteigert und anschließend in gleicher Weise wieder auf den Nullpunkt gesenkt wird.

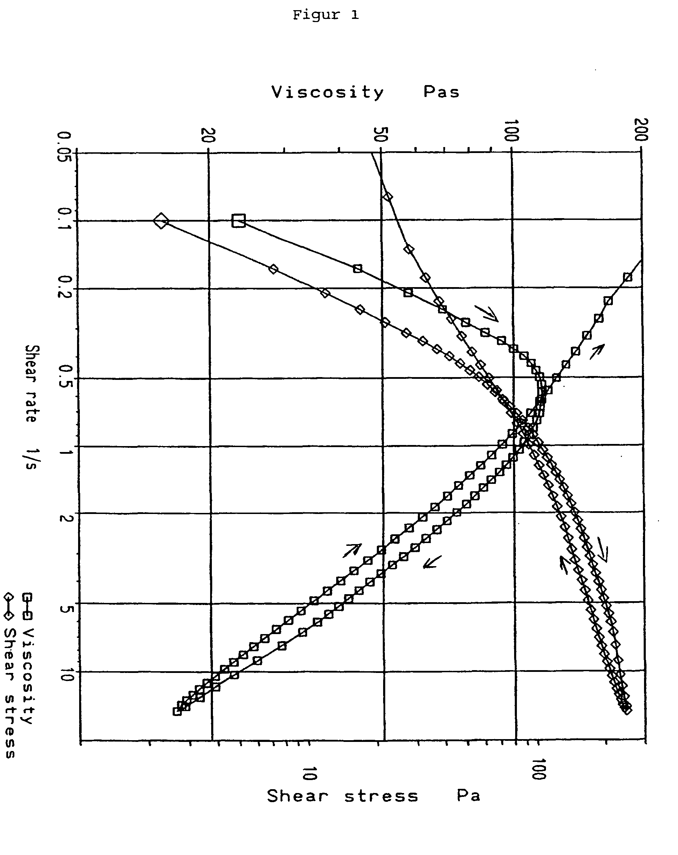

Figur 1 zeigt ein typisches Beispiel eines Messdiagramms, aufgenommen mit einem Bohlin CS System Rheometer bei einer Temperatur von 25°C für das Gel gemäß Beispiel 2. Aufgetragen sind die Viskosität (viscosity, linke Achse, Messwerte dargestellt als Quadrate) und die Scherspannung (shear stress, rechte Achse, Messwerte dargestellt als Rauten) gegen die Schergeschwindigkeit (shear rate). Die Pfeile an den Kurven geben die Richtung der Änderung der Schergeschwindigkeit an. Bei einer Schergeschwindigkeit von ca. 0,6 s$^{-1}$ hat die Viskositätskurve ein Maximum und die Fließkurve (shear stress) einen Wendepunkt. Bei einer Schergeschwindigkeit von ca. 0,7 s$^{-1}$ schneiden sich die Hin- und die Rückkurven.

Die Viskosität des Gels beträgt vorzugsweise von 1000 bis 100.000, besonders bevorzugt von 2000 bis 50.000 mPa s, ganz besonders bevorzugt von 2.500 bis 15.000 mPa s, gemessen als dynamische Viskositätsmessung mit einem HAAKE VT-550 Rheometer, Messkörper SV-DIN bei einer Temperatur von 25°C und einer Schergeschwindigkeit von 50 s$^{-1}$.

Das erfindungsgemäße Mittel kann darüber hinaus die für Haarbehandlungsmittel üblichen Zusatzbestandteile enthalten, z.B. Netzmittel oder Emulgatoren aus den Klassen der nichtionischen, anionischen, kationischen oder amphoteren oberflächenaktiven Tenside, wie Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,1 bis 1 Gew.%; Trübungsmittel, wie z.B. Ethylenglykoldistearat, in einer Menge von etwa 0,2 bis 5,0 Gew.%; Perlglanzmittel, wie z.B. ein Gemisch aus Fettsäuremonoalkylolamid und Ethylenglykoldistearat, in einer Menge von etwa 1,0 bis 10 Gew.%; bakterizide und fungizide Wirkstoffe wie z.B. 2,4,4-Trichlor-2-hydroxydiphenylether oder Methylchlorisothiazolion, in einer Menge von 0,01 bis 1,0 Gew.%; Verdickungsmittel, wie beispielsweise Kokosfettsäurediethanolamid, in einer Menge von etwa 0,2 bis 3,0 Gew.%, Puffersubstanzen, wie beispielsweise Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 4 Gew.%; Fettalkohole, Glanzgeber, Vitamine, Weichmacher, Kämmbarkeitsverbesserer, rückfettende Agenzien und Entschäumer.

Das erfindungsgemäße Mittel wird vorzugsweise in Form eines klaren, transparenten oder zumindest durchscheinenden Gels eingesetzt, wobei das Gel farblos oder eingefärbt sein kann.

Das erfindungsgemäße Mittel zeichnet sich durch seine besonderen rheologischen Eigenschaften aus, die sich insbesondere auch in einer attraktiven, besonders ansprechenden Haptik manifestieren. Das Mittel läßt sich aus einer Verpackung in Form einer Tube, eines Tiegels oder einer Dose im Unterschied zu herkömmlichen, in Tuben verpackten Gelen einfacher und rückstandsfreier entnehmen. Die Gele lassen sich regelrecht abreissen, ohne dass etwas herunter- oder nachläuft. Das Mittel ist gut auf Haaren verteilbar. Als Haarbehandlungsmittel wirkt es nicht belastend auf das Haar und ist daher insbesondere auch für feines Haar geeignet. Als haarfestigendes Mittel bewirkt es eine gute Haltbarkeit der Frisur, ohne dass das Haar verklebt oder belastet wird. Feines Haar bekommt Fülle und Volumen. Als haarfestigendes Gel zeigt das Haarbehandlungsmittel bessere festigende Eigenschaften und insbesondere einen deutlich stärkeren Glanz als herkömmliche Gele auf Carbomer-Basis.

In einer besonderen Ausführungsform ist das erfindungsgemäße Gel zur gleichzeitigen Festigung und temporären Haarfärbung geeignet und enthält zusätzlich mindestens ein temporär haarfärbendes Pigment. Unter temporärer Haarfärbung wird eine Farbänderung von menschlichen Haaren verstanden, die bis zur nächsten Haarwäsche hält und durch Waschen der Haare mit üblichen Shampoos wieder entfernt werden kann. Die Pigmente sind vorzugsweise in einer Menge von 0,01 bis 25 Gew.%, besonders bevorzugt in einer Menge von 5 bis 15 Gew.% enthalten. Bei den Pigmenten handelt es sich vorzugsweise nicht um Nano- sonder um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnung Rona® , Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

Die nachfolgenden Beispiele sollen den Gegenstand der Erfindung näher erläutern. Die in den Beispielen angegebenen Polymergehalte beziehen sich jeweils auf den Feststoffgehalt.

**Beispiele**

| Beispiel 1: Haargel | | |
|---|---|---|
| | A | B |
| PVP/VA Copolymer | 8,0 g | 8,0 g |
| Acrylates/Ceteth-20 Itaconate Copolymer | 3,0 g | - |
| Carbomer | - | 0,5 g |
| Propylenglykol | 2,7 g | 2,7 g |
| Aminomethylpropanol | 1,425 g | 0,5 g |
| Wasser | Ad 100 g | Ad 100 g |

Es wurden zwei Haargele vergleichbarer Viskosität hergestellt und hinsichtlich des Haarglanzes und der Haarfestigung verglichen. Gel A ist erfindungsgemäß, Gel B ist ein herkömmliches Gel auf Carbomer-Basis. Zur Glanzmessung wurden auf Haarsträhnen ä jeweils 2 g Haar jeweils 0,5 g Gel aufgetragen. Die Strähnen wurden über Nacht bei 20°C und 65% relativer Luftfeuchtigkeit getrocknet. Die Glanzmessung erfolgte, indem ein paralleles Lichtbündel auf die Probenoberfläche gerichtet und die Winkelverteilung des reflektierten und des an oder unter der Oberfläche gestreuten Lichts gemessen wurde. Je höher der Anteil des direkt reflektierten und je niedriger der Anteil des gestreuten Lichts ist, desto höher ist der Glanz. Eine Glanzklassifikation kann durch Messung und Bewertung der Winkelverteilung des von der Oberfläche zurückgeworfenen Lichtes erfolgen. Dabei bedeutet eine schmale Winkelverteilung (geringe Halbwertsbreite HWB) stärkeren Glanz und eine breite Winkelverteilung (größere HWB) geringeren Glanz. Die Messung der Winkelverteilung erfolgt mit einer Digitalkamera, die Daten werden in einen Computer eingelesen und mit einer Bildverarbeitungssoftware (Optimate 5.2) ausgewertet. Pro Muster wurden 6 Messungen durchgeführt und der Mittelwert gebildet.

| Unbehandelte Strähnen | HWB = 40° |
|---|---|
| Muster A | HWB = 35° |
| Muster B | HWB = 37° |

Der Austausch des Verdickers führt überraschenderweise zu einer Erhöhung des Haarglanzes. Die Messergebnisse korrelieren mit einer visuellen Glanzbeurteilung der Strähnen durch ein in der Beurteilung von Haarqualitäten erfahrenes Expertenpanel.

Der Vergleich der Festigungsleistung erfolgte durch Messung der Lockenstabilität (Curl Retention CR). Hierfür wurden die Gele auf Haarsträhnen aufgetragen. Die Strähnen wurden nach einer Einwirkzeit von 10 Minuten auf Spiralwickler gewickelt, 30 min bei 70°C und mindestens eine Stunde bei 20°C und 85% relativer Luftfeuchte gelagert. Anschließend wurden die Strähnen von den Wicklern abgewickelt, mit 50 mg Gewichten beschwert und ausgehängt.

Nach 5 Stunden und nach 24 Stunden wurde die Länge der Strähnen gemessen und die Lockenstabilität berechnet:

$$CR = (L_0 - L_t) / (L_0 - L_1) * 100\%$$

$L_0$ : Länge der gestreckten Strähne
$L_t$ : Länge der ausgehängten Locke
$L_1$ : Länge der aufgewickelten Strähne

Pro Muster wurde eine 3fach-Messung durchgeführt und der Mittelwert gebildet.

| Unbehandelte Strähnen | CR (5h) = 42% | CR (24h) = 21% |
|---|---|---|
| Muster A | CR (5h) = 75% | CR (24h) = 60% |
| Muster B | CR (5h) = 47% | CR (24h) = 29% |

Der Austausch des Verdickers führt zu einer überraschend hohen Lockenstabilität.

| **Beispiel 2:** Haargel | |
|---|---|
| PVP/VA Copolymer | 1,0 g |
| Acrylates/Ceteth-20 Itaconate Copolymer | 3,0 g |
| Aminomethylpropanol | 1,6 g |
| Wasser | ad 100 g |
| Viskosität ca. 6550 mPa s (25°C) | |

In Abbildung 1 sind die Fließ- und Viskositätskurven bei im Bereich von 0 bis 20 $s^{-1}$ zu- und abnehmender Schergeschwindigkeit dargestellt.

| **Beispiel 3:** Haargel | |
|---|---|
| PVP/VA Copolymer | 2,0 g |
| Acrylates/Ceteth-20 Itaconate Copolymer | 1,5 g |
| Aminomethylpropanol | 0,8 g |
| PEG-40 Hydrogenated Castor Oil | 0,3 g |
| Parfüm | 0,2 g |
| Wasser | ad 100 g |
| Viskosität ca. 2600 mPa s (25°C) | |

| **Beispiel 4:** Haargel | |
|---|---|
| VA/Crotonates Copolymer (Luviset® 66) | 1,0 g |
| Acrylates/Ceteth-20 Itaconate Copolymer | 2,25 g |
| Aminomethylpropanol | 1,4 g |
| PEG-40 Hydrogenated Castor Oil | 0,3 g |
| Parfüm | 0,2 g |
| Wasser | ad 100 g |
| Viskosität ca. 5800 mPa s (25°C) | |

| Beispiel 5: Haargel mit Farbpigment | |
|---|---|
| VA/Crotonates Copolymer (Luviset® 66) | 1,0 g |
| Acrylates/Ceteth-20 Itaconate Copolymer | 2,25 g |
| Aminomethylpropanol | 1,4 g |
| Polysorbate 40 | 0,8 g |
| Timiron® Gold Plus MP-25 | 0,2 g |
| Wasser | ad 100 g |
| Viskosität ca. 5600 mPa s (25°C) | |

| Beispiel 6: Haargel | |
|---|---|
| Octylacrylamide/Acrylates/Butylaminoethyl Methacrylate Copolymer (Amphomer®) | 2,5 g |
| Acrylates/Ceteth-20 Itaconate Copolymer | 1,5 g |
| Aminomethylpropanol | 1,23 g |
| FD&C Green No. 3 (CI 42053) | 0,1 g |
| Wasser | ad 100 g |
| Viskosität ca. 3.600 mPa s (25°C) | |

| Beispiel 7: Haargel | |
|---|---|
| Polyvinylpyrrolidon K80 | 2,0 g |
| Acrylates/Ceteth-20 Itaconate Copolymer | 3,75 g |
| Aminomethylpropanol | 2,0 g |
| Polysorbate 40 | 0,8 g |
| Parfüm | 0,1 g |
| Wasser | ad 100 g |
| Viskosität ca. 14200 mPa s (25°C) | |

**Patentansprüche**

**Patentansprüche für folgende Vertragsstaaten : AT, BE, CH, CY, DK, ES, GB, GR, IE, IT, LI, LU, MC, NL, PT, SE, TR**

**1.** Haarbehandlungsmittel in Gelform auf wässriger Basis mit einer Viskosität von mindestens 1000 mPa s (gemessen bei 25°C und einer Schergeschwindigkeit von 50 s$^{-1}$), einem pH-Wert größer als 7 und mit einem Gehalt an

(A) mindestens 0,5 Gew.% eines Copolymeren aus einer ersten Monomerart, ausgewählt aus Itaconsäure-monoestern der allgemeinen Formel $CH_2=C(COOR^1)CH_2COOR^2$ wobei einer der Substituenten $R^1$ und $R^2$ für Wasserstoff und der andere für die Gruppe $-(CH_2CH_2O)_x-R^3$ steht, x eine Zahl zwischen 1 und 100 bedeutet und $R^3$ eine Cetylgruppe bedeutet und einer zweiten Monomerart, ausgewählt aus Acrylat-Monomeren und (B) einem Gehalt an mindestens 0,1 Gew.% mindestens eines haarfestigenden nichtionischen, anionischen, zwitterionischen oder amphoteren Polymers.

ausgenommen ein Gel mit einem Gehalt an einer Kombination von (a) Acrylates/Ceteth-20-itaconat Copolymer, (b) Celluloseacetatphtalat und (c) $HO-(CH_2CH_2O)_{75}-(CH(CH_3)CH_2O)_{30}-(CH_2CH_2O)_{75}-H$.

**2.** Mittel nach Anspruch 1, **dadurch gekennzeichnet, dass** das Copolymer (A) in einer Menge von 1,5 bis 10 Gew.

% vorliegt.

**3.** Mittel nach Anspruch 1 oder 2, **dadurch gekennzeichnet, dass** das Polymer (B) in einer Menge von 0,1 bis 15 Gew.% vorliegt.

**4.** Mittel nach einem der vorhergehenden Ansprüche, **dadurch gekennzeichnet, dass** x für eine Zahl von 10 bis 40 steht.

**5.** Mittel nach einem der vorhergehenden Ansprüche, **dadurch gekennzeichnet, dass** die zweite Monomerart des Copolymers (A) ausgewählt ist aus Acrylsäure, Methacrylsäure, Acrylsäurealkylestern und Methacrylsäurealkylestern, wobei die Alkylgruppen 1 bis 10 C-Atome aufweisen.

**6.** Mittel nach einem der vorhergehenden Ansprüche, **dadurch gekennzeichnet, dass** das haarfestigende Polymer (B) ausgewählt ist aus Polyvinylpyrrolidon, Vinylpyrrolidon/Vinylacetat Copolymer, Vinylacetat/Crotonsäure Copolymeren, Terpolymeren aus Vinylacetat, Crotonat und Vinylalkanoat, partialveresterten Copolymeren zwischen Vinylmethylether und Maleinsäureanhydrid, Copolymeren aus Acryl- oder Methacrylsäure mit Alkylacrylaten und/ oder N-Alkylacrylamiden und Polystyrolsulfonaten, Copolymeren aus Alkylacrylamid, Alkylaminoalkylmethacrylat und zwei oder mehr Monomeren ausgewählt aus Acrylsäure, Methacrylsäure oder deren C1- bis C4-Alkylestern, wobei mindestens eines der Monomere eine Säuregruppe aufweist.

**7.** Mittel nach einem der vorhergehenden Ansprüche, **dadurch gekennzeichnet, dass** es im wesentlichen frei ist von kationischen Polymeren.

**8.** Mittel nach einem der vorhergehenden Ansprüche, **dadurch gekennzeichnet, dass** es zusätzlich mindestens einen Alkohol enthält ausgewählt aus einwertigen Alkoholen mit 1 bis 4 C-Atomen und mehrwertigen Alkoholen mit 2 bis 5 C-Atomen.

**9.** Mittel nach einem der vorhergehenden Ansprüche, **dadurch gekennzeichnet, dass** es zusätzlich mindestens ein haarfärbendes Pigment enthält.

**10.** Mittel nach Anspruch 9, **dadurch gekennzeichnet, dass** die Pigmente zu 0,01 bis 25 Gew.% enthalten sind und ausgewählt sind aus Metalloxiden, Ultramarin, Glanzpigmenten, Metalleffekt-Pigmenten, Perlglanzpigmenten, Fluoreszenzpigmenten, Phosphoreszenzpigmenten, Metallhydroxiden, Metalloxidhydraten, Mischphasenpigmenten, schwefelhaltigen Silicaten, Metallsulfiden, komplexen Metallcyaniden, Metallsulfaten, Metallchromaten, Metallmolybdaten, Bronzepigmenten, Carmine, Pigmenten auf Mica- oder Glimmerbasis, welche mit einem Metalloxid oder einem Metalloxychlorid sowie gegebenenfalls weiteren farbgebenden Stoffen wie Eisenoxiden, Eisenblau, Ultramarine, Carmine etc. beschichtet sind und/oder wobei die Farbe durch Variation der Schichtdicke bestimmt ist.

**Patentansprüche für folgenden Vertragsstaat : DE**

**1.** Haarbehandlungsmittel in Gelform auf wässriger Basis mit einer Viskosität von mindestens 1000 mPa s (gemessen bei 25°C und einer Schergeschwindigkeit von 50 s$^{-1}$), einem pH-Wert größer als 7 und mit einem Gehalt an

(A) mindestens 0,5 Gew.% eines Copolymeren aus einer ersten Monomerart, ausgewählt aus Itaconsäuremonoestern der allgemeinen Formel $CH_2=C(COOR^1)CH_2COOR^2$ wobei einer der Substituenten $R^1$ und $R^2$ für Wasserstoff und der andere für die Gruppe -$(CH_2CH_2O)_x$-$R^3$ steht, x eine Zahl zwischen 1 und 100 bedeutet und $R^3$ eine Cetylgruppe bedeutet und einer zweiten Monomerart, ausgewählt aus Acrylat-Monomeren und (B) einem Gehalt an mindestens 0,1 Gew.% mindestens eines haarfestigenden nichtionischen, anionischen, zwitterionischen oder amphoteren Polymers.

**2.** Mittel nach Anspruch 1, **dadurch gekennzeichnet, dass** das Copolymer (A) in einer Menge von 1,5 bis 10 Gew. % vorliegt.

**3.** Mittel nach Anspruch 1 oder 2, **dadurch gekennzeichnet, dass** das Polymer (B) in einer Menge von 0,1 bis 15 Gew.% vorliegt.

**4.** Mittel nach einem der vorhergehenden Ansprüche, **dadurch gekennzeichnet, dass** x für eine Zahl von 10 bis 40 steht.

**5.** Mittel nach einem der vorhergehenden Ansprüche, **dadurch gekennzeichnet, dass** die zweite Monomerart des Copolymers (A) ausgewählt ist aus Acrylsäure, Methacrylsäure, Acrylsäurealkylestern und Methacrylsäurealkylestern, wobei die Alkylgruppen 1 bis 10 C-Atome aufweisen.

**6.** Mittel nach einem der vorhergehenden Ansprüche, **dadurch gekennzeichnet, dass** das haarfestigende Polymer (B) ausgewählt ist aus Polyvinylpyrrolidon, Vinylpyrrolidon/Vinylacetat Copolymer, Vinylacetat/Crotonsäure Copolymeren, Terpolymeren aus Vinylacetat, Crotonat und Vinylalkanoat, partialveresterten Copolymeren zwischen Vinylmethylether und Maleinsäureanhydrid, Copolymeren aus Acryl- oder Methacrylsäure mit Alkylacrylaten und/ oder N-Alkylacrylamiden und Polystyrolsulfonaten, Copolymeren aus Alkylacrylamid, Alkylaminoalkylmethacrylat und zwei oder mehr Monomeren ausgewählt aus Acrylsäure, Methacrylsäure oder deren C1- bis C4-Alkylestern, wobei mindestens eines der Monomere eine Säuregruppe aufweist.

**7.** Mittel nach einem der vorhergehenden Ansprüche, **dadurch gekennzeichnet, dass** es im wesentlichen frei ist von kationischen Polymeren.

**8.** Mittel nach einem der vorhergehenden Ansprüche, **dadurch gekennzeichnet, dass** es zusätzlich mindestens einen Alkohol enthält ausgewählt aus einwertigen Alkoholen mit 1 bis 4 C-Atomen und mehrwertigen Alkoholen mit 2 bis 5 C-Atomen.

**9.** Mittel nach einem der vorhergehenden Ansprüche, **dadurch gekennzeichnet, dass** es zusätzlich mindestens ein haarfärbendes Pigment enthält.

**10.** Mittel nach Anspruch 9, **dadurch gekennzeichnet, dass** die Pigmente zu 0,01 bis 25 Gew.% enthalten sind und ausgewählt sind aus Metalloxiden, Ultramarin, Glanzpigmenten, Metalleffekt-Pigmenten, Perlglanzpigmenten, Fluoreszenzpigmenten, Phosphoreszenzpigmenten, Metallhydroxiden, Metalloxidhydraten, Mischphasenpigmenten, schwefelhaltigen Silicaten, Metallsulfiden, komplexen Metallcyaniden, Metallsulfaten, Metallchromaten, Metallmolybdaten, Bronzepigmenten, Carmine, Pigmenten auf Mica- oder Glimmerbasis, welche mit einem Metalloxid oder einem Metalloxychlorid sowie gegebenenfalls weiteren farbgebenden Stoffen wie Eisenoxiden, Eisenblau, Ultramarine, Carmine etc. beschichtet sind und/oder wobei die Farbe durch Variation der Schichtdicke bestimmt ist.

**Claims**

**Claims for the following Contracting States : AT, BE, CH, CY, DK, ES, GB, GR, IE, IT, LI, LU, MC, NL, PT, SE, TR**

**1.** Aqueous-based hair-treatment agent in gel form with a viscosity of at least 1000 mPa s (measured at 25°C and a shear velocity of 50 s$^{-1}$), a pH greater than 7 and with a content of

(A) at least 0.5% by weight of a copolymer of a first type of monomer chosen from itaconic acid monoesters of the general formula $CH_2=C(COOR^1)CH_2COOR^2$, where one of the substituents $R^1$ and $R^2$ is hydrogen and the other is the group $-(CH_2CH_2O)_x-R^3$, x is a number between 1 and 100, and $R^3$ is a cetyl group, and a second type of monomer chosen from acrylate monomers and
(B) a content of at least 0.1% by weight of at least one hair-setting nonionic, anionic, zwitterionic or amphoteric polymer.

**2.** Agent according to Claim 1, **characterized in that** the copolymer (A) is present in an amount of from 1.5 to 10% by weight.

**3.** Agent according to Claim 1 or Claim 2, **characterized in that** the polymer (B) is present in an amount of from 0.1 to 15% by weight.

**4.** Agent according to one of the preceding claims, **characterized in that** x is a number from 10 to 40.

**5.** Agent according to one of the preceding claims, **characterized in that** the second type of monomer of the copolymer (A) is chosen from acrylic acid, methacrylic acid, acrylic acid alkyl esters and methacrylic acid alkyl esters, where the alkyl groups have 1 to 10 carbon atoms.

6. Agent according to one of the preceding claims, **characterized in that** the hair-setting polymer (B) is chosen from polyvinylpyrrolidone, vinylpyrrolidone/vinyl acetate copolymer, vinyl acetate/crotonic acid copolymers, terpolymers of vinyl acetate, crotonate and vinyl alkanoate, partially esterified copolymers between vinyl methyl ether and maleic anhydride, copolymers of acrylic or methacrylic acid with alkyl acrylates and/or N-alkylacrylamides and polystyrenesulphonates, copolymers of alkylacrylamide, alkylaminoalkyl methacrylate and two or more monomers chosen from acrylic acid, methacrylic acid or $C_1$-$C_4$-alkyl esters thereof, where at least one of the monomers has an acid group.

7. Agent according to one of the preceding claims, **characterized in that** it is essentially free from cationic polymers.

8. Agent according to one of the preceding claims, **characterized in that** it additionally comprises at least one alcohol chosen from monohydric alcohols having 1 to 4 carbon atoms and polyhydric alcohols having 2 to 5 carbon atoms.

9. Agent according to one of the preceding claims, **characterized in that** it additionally comprises at least one hair-colouring pigment.

10. Agent according to Claim 9, **characterized in that** the pigments are present in amounts of from 0.01 to 25% by weight and are chosen from metal oxides, ultramarine, lustre pigments, metal effect pigments, pearlescent pigments, fluorescent pigments, phosphorescent pigments, metal hydroxides, metal oxide hydrates, mixed phase pigments, sulphur-containing silicates, metal sulphides, complex metal cyanides, metal sulphates, metal chromates, metal molybdates, bronze pigments, carmine, pigments based on mica which are coated with a metal oxide or a metal oxychloride and optionally further colour-imparting substances such as iron oxides, Prussian blue, ultramarine, carmine etc. and/or where the colour is determined by varying the layer thickness.


**Claims for the following Contracting State : DE**

1. Aqueous-based hair-treatment agent in gel form with a viscosity of at least 1000 mPa s (measured at 25°C and a shear velocity of 50 s$^{-1}$), a pH greater than 7 and with a content of

(A) at least 0.5% by weight of a copolymer of a first type of monomer chosen from itaconic acid monoesters of the general formula $CH_2=C(COOR^1)CH_2COOR^2$, where one of the substituents $R^1$ and $R^2$ is hydrogen and the other is the group -$(CH_2CH_2O)_x$-$R^3$, x is a number between 1 and 100, and $R^3$ is a cetyl group, and a second type of monomer chosen from acrylate monomers and -
(B) a content of at least 0.1% by weight of at least one hair-setting nonionic, anionic, zwitterionic or amphoteric polymer

excluding a gel with a content of a combination of
(a) acrylates/ceteth-20 itaconate copolymer, (b) cellulose acetate phthalate and
(c) $HO-(CH_2CH_2O)_{75}-(CH(CH_3)CH_2O)_{30}-(CH_2CH_2O)_{75}-H$.

2. Agent according to Claim 1, **characterized in that** the copolymer (A) is present in an amount of from 1.5 to 10% by weight.

3. Agent according to Claim 1 or Claim 2, **characterized in that** the polymer (B) is present in an amount of from 0.1 to 15% by weight.

4. Agent according to one of the preceding claims, **characterized in that** x is a number from 10 to 40.

5. Agent according to one of the preceding claims, **characterized in that** the second type of monomer of the copolymer (A) is chosen from acrylic acid, methacrylic acid, acrylic acid alkyl esters and methacrylic acid alkyl esters, where the alkyl groups have 1 to 10 carbon atoms.

6. Agent according to one of the preceding claims, **characterized in that** the hair-setting polymer (B) is chosen from polyvinylpyrrolidone, vinylpyrrolidone/vinyl acetate copolymer, vinyl acetate/crotonic acid copolymers, terpolymers of vinyl acetate, crotonate and vinyl alkanoate, partially esterified copolymers between vinyl methyl ether and maleic anhydride, copolymers of acrylic or methacrylic acid with alkyl acrylates and/or N-alkylacrylamides and polystyrenesulphonates, copolymers of alkylacrylamide, alkylaminoalkyl methacrylate and two or more monomers chosen from acrylic acid, methacrylic acid or $C_1$-$C_4$-alkyl esters thereof, where at least one of the monomers has an acid group.

7. Agent according to one of the preceding claims, **characterized in that** it is essentially free from cationic polymers.

8. Agent according to one of the preceding claims, **characterized in that** it additionally comprises at least one alcohol chosen from monohydric alcohols having 1 to 4 carbon atoms and polyhydric alcohols having 2 to 5 carbon atoms.

9. Agent according to one of the preceding claims, **characterized in that** it additionally comprises at least one hair-colouring pigment.

10. Agent according to Claim 9, **characterized in that** the pigments are present in amounts of from 0.01 to 25% by weight and are chosen from metal oxides, ultramarine, lustre pigments, metal effect pigments, pearlescent pigments, fluorescent pigments, phosphorescent pigments, metal hydroxides, metal oxide hydrates, mixed phase pigments, sulphur-containing silicates, metal sulphides, complex metal cyanides, metal sulphates, metal chromates, metal molybdates, bronze pigments, carmine, pigments based on mica which are coated with a metal oxide or a metal oxychloride and optionally further colour-imparting substances such as iron oxides, Prussian blue, ultramarine, carmine etc. and/or where the colour is determined by varying the layer thickness.

**Revendications**

**Revendications pour les Etats contractants suivants : AT, BE, CH, CY, DK, ES, GB, GR, IE, IT, LI, LU, MC, NL, PT, SE, TR**

1. Composition de traitement capillaire sous forme de gel à base aqueuse ayant une viscosité d'au moins 1 000 mPa. s (mesurée à 25°C et à une vitesse de cisaillement de 50 s$^{-1}$), un pH supérieur à 7 et ayant une teneur

   (A) d'au moins 0,5 % en poids en un polymère à base d'un premier type de monomère, choisi parmi les monoesters d'acide itaconique de formule générale $CH_2$=C(COOR$^1$)CH$_2$COOR$^2$, dans laquelle l'un des substituants R$^1$ et R$^2$ représente un atome d'hydrogène et l'autre représente le groupe - (CH$_2$CH$_2$O)$_x$-R$^3$, x représente un nombre compris entre 1 et 100, et R$^3$ représente un groupe cétyle, et d'un second type de monomère choisi parmi les monomères acrylate et
   (B) une teneur d'au moins 0,1 % en poids en au moins un polymère non ionique, anionique, zwitterionique ou amphotère, fixant les cheveux.

2. Composition selon la revendication 1, **caractérisée en ce que** le copolymère (A) est présent en une quantité de 1,5 à 10 % en poids.

3. Composition selon la revendication 1 ou 2, **caractérisée en ce que** le copolymère (B) est présent en une quantité de 0,1 à 15 % en poids.

4. Composition selon l'une quelconque des revendications précédentes, **caractérisée en ce que** x représente un nombre allant de 10 à 40.

5. Composition selon l'une quelconque des revendications précédentes, **caractérisée en ce que** le second type de monomère du copolymère (A) est choisi parmi l'acide acrylique, l'acide méthacrylique, des esters alkyliques d'acide acrylique et des esters alkyliques d'acide méthacrylique, les groupes alkyle ayant de 1 à 10 atomes de carbone.

6. Composition selon l'une quelconque des revendications précédentes, **caractérisée en ce que** le polymère (B) fixant les cheveux est choisi parmi la polyvinylpyrrolidone, un copolymère vinylpyrrolidone/acétate de vinyle, des copolymères acétate de vinyle/acide crotonique, des terpolymères d'acétate de vinyle, crotonate et alcanoate de vinyle, des copolymères entre l'éther vinylméthylique et l'anhydride maléique, partiellement estérifiés, des copolymères d'acide acrylique ou méthacrylique avec des acrylates d'alkyle et/ou des N-alkylacrylamides et des polystyrènesulfonates, des copolymères d'alkylacrylamide, méthacrylate d'alkylaminoalkyle et de deux ou plus de deux monomères choisis parmi l'acide acrylique, l'acide méthacrylique ou leurs esters alkyliques en $C_1$-$C_4$, au moins l'un des monomères comportant un groupe acide.

**7.** Composition selon l'une quelconque des revendications précédentes, **caractérisée en ce qu'**elle est pratiquement exempte de polymères cationiques.

**8.** Composition selon l'une quelconque des revendications précédentes, **caractérisée en ce qu'**additionnellement elle contient au moins un alcool choisi parmi les alcools monohydriques ayant de 1 à 4 atomes de carbone et les alcools polyhydriques ayant de 2 à 5 atomes de carbone.

**9.** Composition selon l'une quelconque des revendications précédentes, **caractérisée en ce qu'**additionnellement elle contient au moins un pigment colorant les cheveux.

**10.** Composition selon la revendication 9, **caractérisée en ce que** les pigments sont contenus à raison de 0,01 à 25 % en poids et sont choisis parmi des oxydes métalliques, l'outremer, des pigments brillants, des pigments à effet métallique, des pigments nacrés, des pigments fluorescents, des pigments phosphorescents, des hydroxydes de métaux, des hydrates de métaux, des pigments en phases mixtes, des silicates soufrés, des sulfures métalliques, des cyanures métalliques complexes, des sulfates métalliques, des chromates métalliques, des molybdates métalliques, des pigments bronze, des carmins, des pigments à base de mica qui sont enrobés d'un oxyde métallique ou d'un oxychlorure métallique ainsi qu'éventuellement d'autres substances colorantes telles que des oxydes de fer, le bleu de Prusse, des outremers, des carmins, etc., la couleur étant déterminée par une variation de l'épaisseur de couche.

**Revendications pour l'Etat contractant suivant : DE**

**1.** Composition de traitement capillaire sous forme de gel à base aqueuse ayant une viscosité d'au moins 1 000 mPa. s (mesurée à 25°C et à une vitesse de cisaillement de 50 s$^{-1}$), un pH supérieur à 7 et ayant une teneur

(A) d'au moins 0,5 % en poids en un polymère à base d'un premier type de monomère, choisi parmi les monoesters d'acide itaconique de formule générale $CH_2=C(COOR^1)CH_2COOR^2$, dans laquelle l'un des substituants $R^1$ et $R^2$ représente un atome d'hydrogène et l'autre représente le groupe - $(CH_2CH_2O)_x$-$R^3$, x représente un nombre compris entre 1 et 100, et $R^3$ représente un groupe cétyle, et d'un second type de monomère choisi parmi les monomères acrylate et

(B) une teneur d'au moins 0,1 % en poids en au moins un polymère non ionique, anionique, zwitterionique ou amphotère, fixant les cheveux, à l'exception d'un gel ayant une teneur d'une combinaison de (a) copolymère acrylates/itaconate de Ceteth-20, (b) phtalate d'acétate cellulose et (c) HO-$(CH_2CH_2O)_{75}$-$(CH(CH_3)$ $CH_2O)_{30}$-$(CH_2CH_2O)_{75}$-H.

**2.** Composition selon la revendication 1, **caractérisée en ce que** le copolymère (A) est présent en une quantité de 1,5 à 10 % en poids.

**3.** Composition selon la revendication 1 ou 2, **caractérisée en ce que** le copolymère (B) est présent en une quantité de 0,1 à 15 % en poids.

**4.** Composition selon l'une quelconque des revendications précédentes, **caractérisée en ce que** x représente un nombre allant de 10 à 40.

**5.** Composition selon l'une quelconque des revendications précédentes, **caractérisée en ce que** le second type de monomère du copolymère (A) est choisi parmi l'acide acrylique, l'acide méthacrylique, des esters alkyliques d'acide acrylique et des esters alkyliques d'acide méthacrylique, les groupes alkyle ayant de 1 à 10 atomes de carbone.

**6.** Composition selon l'une quelconque des revendications précédentes, **caractérisée en ce que** le polymère (B) fixant les cheveux est choisi parmi la polyvinylpyrrolidone, un copolymère vinylpyrrolidone/acétate de vinyle, des copolymères acétate de vinyle/acide crotonique, des terpolymères d'acétate de vinyle, crotonate et alcanoate de vinyle, des copolymères entre l'éther vinylméthylique et l'anhydride maléique, partiellement estérifiés, des copolymères d'acide acrylique ou méthacrylique avec des acrylates d'alkyle et/ou des N-alkylacrylamides et des polystyrènesulfonates, des copolymères d'alkylacrylamide, méthacrylate d'alkylaminoalkyle et de deux ou plus de deux monomères choisis parmi l'acide acrylique, l'acide méthacrylique ou leurs esters alkyliques en $C_1$-$C_4$, au moins l'un des monomères comportant un groupe acide.

7. Composition selon l'une quelconque des revendications précédentes, **caractérisée en ce qu'**elle est pratiquement exempte de polymères cationiques.

8. Composition selon l'une quelconque des revendications précédentes, **caractérisée en ce qu'**additionnellement elle contient au moins un alcool choisi parmi les alcools monohydriques ayant de 1 à 4 atomes de carbone et les alcools polyhydriques ayant de 2 à 5 atomes de carbone.

9. Composition selon l'une quelconque des revendications précédentes, **caractérisée en ce qu'**additionnellement elle contient au moins un pigment colorant les cheveux.

10. Composition selon la revendication 9, **caractérisée en ce que** les pigments sont contenus à raison de 0,01 à 25 % en poids et sont choisis parmi des oxydes métalliques, l'outremer, des pigments brillants, des pigments à effet métallique, des pigments nacrés, des pigments fluorescents, des pigments phosphorescents, des hydroxydes de métaux, des hydrates de métaux, des pigments en phases mixtes, des silicates soufrés, des sulfures métalliques, des cyanures métalliques complexes, des sulfates métalliques, des chromates métalliques, des molybdates métalliques, des pigments bronze, des carmins, des pigments à base de mica qui sont enrobés d'un oxyde métallique ou d'un oxychlorure métallique ainsi qu'éventuellement d'autres substances colorantes telles que des oxydes de fer, le bleu de Prusse, des outremers, des carmins, etc., la couleur étant déterminée par une variation de l'épaisseur de couche.

Figur 1